# EUROPEAN PATENT APPLICATION

(11) **EP 4 458 292 A2**
(43) Date of publication of application: **06.11.2024**
(21) Application number: 24201859.6
(22) Date of filing: 19.05.2022
(51) Int. Cl.: A61B 17/34

(54) **TISSUE PUNCTURE SYSTEM WITH STEERABLE MICROCATHETER AND ELECTRICALLY CONDUCTIVE GUIDEWIRE**

(30) Priority: 20.05.2021 US 202163190865 P; 13.05.2022 US 202217743852
(62) Divisional of application: 22174281.0
(71) Applicant: Biosense Webster (Israel) Ltd., 2066717 Yokneam (IL)
(72) Inventor: HIGHSMITH, Debby, Irvine, 92618 (US)
(74) Representative: Carpmaels & Ransford LLP

(57) **Abstract**

A microcatheter with a guidewire therein can be steered to target tissue, then the target tissue can be punctured with the guidewire to create a transseptal puncture. The microcatheter can have a diameter substantially smaller than known sheaths which are typically used to guide a needle to a target puncture site in known transseptal puncture treatments. The guidewire can have an atraumatic, electrically conductive distal end that can be electrically energized to puncture the target tissue. Once the guide wire is across, ancillary devices such as a dilator and sheath can be delivered over the guide wire across the transseptal puncture. The microcatheter can include one or more location sensors. A navigation module can use the electrically conductive distal end as a reference electrode to the location sensor(s) of the microcatheter.

## Description

### CROSS REFERENCE TO RELATED APPLICATIONS

This application claims the benefit of priority under 35 U.S.C. § 119 to prior filed U.S. Provisional Patent Application No. 63/190,865 filed on May 20, 2021, which is hereby incorporated by reference as set forth in full herein.

### FIELD

The present invention is directed toward methods and devices for performing diagnostic and/or therapeutic procedures on tissue and organs. More specifically to methods and devices for perforation procedures such as a transseptal perforation procedure.

### BACKGROUND

In medical procedures involving a patient's heart, there are numerous diagnostic and therapeutic procedures that include transseptal left heart catheterization, i.e. catherization through the left atrium. The transseptal approach provides access for both interventional cardiologists who perform antegrade mitral balloon valvuloplasty and for cardiac electrophysiologists who ablate left sided accessory pathways or perform transcatheter atrial-fibrillation therapeutic tactics.

Currently, transseptal puncture can generally involve: (1) positioning a guide wire in the right atrium; (2) advancing a transseptal sheath with dilator therein over the guidewire to the right atrium; (3) delivering a needle through the dilator until a distal end of the needle is inside the dilator a short distance from a distal end of the dilator; (4) manipulating the sheath and dilator until the dilator distal end is pressed against target tissue; (5) moving the distal end of the needle out of the dilator and through the tissue to puncture the tissue; and (6) moving the dilator and sheath through the punctured tissue into the left atrium. In this process, the dilator, transseptal sheath, and/or needle can have some pre-defined curvature at their respective distal ends to aid in positioning of the system for puncture. A procedure using a more simplistic system typically relies on fluoroscopy to visualize the position of the system and mechanical feedback (e.g. feeling a "click" when the dilator tip crosses a tissue ridge) to verify position prior to puncture. In more advanced systems, a sheath having navigation sensors and a distal curvature that can be reshaped during positioning (e.g. CARTO VIZIGO ^{™} bi-directional guiding sheath) can reduce reliance on fluoroscopy and provide greater mechanical control of the system.

U.S. Patent Publication 2004/0220471 (incorporated herein as if disclosed in its entirety and attached in the Appendix to priority application U.S. 63/190,865) discloses a method and device for transseptal facilitation using a location system. During transseptal perforation, once the fossa ovalis is found, a penetrating device such as a HEARTSPAN^{™} transseptal needle is delivered through vasculature to the fossa ovalis via a sheath such as a PREFACE^{®} Sheath; then the penetrating device exits the sheath and punctures the fossa ovalis. The HEARTSPAN^{™} transseptal needle and PREFACE^{®} Sheath are available from Biosense Webster, a Johnson and Johnson company.

While in many current treatments, the needle in the above-described procedure is a mechanical needle with a sharp end, structures which rely on electrical energy to puncture are an option. U.S. Patent Publication 2012/0232546, U.S. Patent 8,235,986, U.S. Patent 10,065,032, and U.S. Provisional Patent Application No. 63/046,266 filed July 7, 2020 (each incorporated herein as if disclosed in their entirety and attached in the Appendix to priority application U.S. 63/190,865) each discloses a respective perforation apparatus which relies on electrical energy for puncture (e.g. radio frequency).

### SUMMARY

A system and method are disclosed herein for transseptal puncture which include steering a microcatheter with a guidewire therein to a target puncture site then puncturing the target puncture site with the guidewire. The microcatheter can have a diameter substantially smaller than known sheaths which are typically used to guide a needle to a target puncture site in known treatments. The guidewire can have an atraumatic, electrically conductive distal end that can be electrically energized to puncture tissue for a transseptal puncture. Once the guide wire is across, ancillary devices such as a dilator and sheath can be delivered over the guide wire across the transseptal puncture. Alternatively, the microcatheter can have a tapered distal end to function as a dilator and the sheath can be delivered directly over the microcatheter. The microcatheter can additionally include one or more location sensors. In some examples a mapping/navigation module can utilize the electrically conductive distal end of the guidewire as a reference electrode to the location sensor(s) of the microcatheter.

An example transseptal puncturing system can include a steerable microcatheter, a guidewire, and a generator. The steerable microcatheter can include an elongated member with lumen extending therethrough to define a longitudinal axis, a deflectable distal portion, and a location sensor disposed proximate the deflectable distal portion. The guidewire can be disposed within the lumen of the microcatheter. The guidewire can have an electrically conductive core, an electrically conductive distal end, an electrically conductive proximal end, and an outer diameter less than and approximately equal to an inner diameter of the lumen of the elongated member of the microcatheter. The generator can be in electrical contact with the electrically conductive proximal end of the guidewire. The generator can be configured to provide electrical energy to the distal end of the guidewire sufficient to puncture tissue so that the tissue is punctured without requiring a sharp end of a needle.

The example transseptal puncturing system can further include a navigation module in electrical connection with the location sensor. The navigation module can be configured to determine a position of the distal end of the microcatheter in the heart based at least in part on the location sensor in reference to the electrically conductive distal end of the guidewire.

The example transseptal puncturing system can further include a dilator and a sheath. The dilator can have a lumen therethrough that has an inner diameter greater than and approximately equal to the inner diameter of the lumen of the microcatheter. The sheath can be configured to advance over the dilator. Alternatively, the microcatheter can have a tapered distal end and function as a dilator. When the system is configured as such, the tapered distal end of the microcatheter can have a distal outer diameter greater than and approximately equal to the outer diameter of the guidewire and a proximal outer diameter less than and approximately equal to the inner diameter of the lumen of the sheath.

The steerable microcatheter can further include one or more pull wires connected to its distal portion. At least one of the pull wires can be pulled to deflect the distal portion with respect to the longitudinal axis.

The example transseptal puncturing system can further include an impedance monitoring module in communication with the generator, in electrical communication with the proximal end of the guidewire, and configured to measure impedance at the distal end of the guidewire. The generator can be configured to provide the electrical energy to the distal end of the guidewire based at least in part on the impedance measured by the impedance monitoring module.

The example transseptal puncturing system can further include a mapping module configured to generate a map of at least a portion of an interatrial septum using the location sensor. The location sensor can include a magnetic coil. Additionally, or alternatively, the location sensor can include an exposed electrode, the system can include one or more body patches, and the navigation module can be configured to determine a position of the distal end of the microcatheter in the heart based at least in part on impedance between the body patches and the exposed electrode of the location sensor.

Another example transseptal puncturing system can include a steerable microcatheter, a guidewire, and a navigation module. The steerable microcatheter can have an elongated tubular member with a lumen extending therethrough to define a longitudinal axis, a deflectable distal portion, and a location sensor disposed proximate the distal portion along the longitudinal axis. The guidewire can have a portion disposed in the lumen of the microcatheter. The guidewire can have an electrically conductive core, an electrically conductive distal end, and an electrically conductive proximal end. The navigation module can be configured to determine a position of the distal end of the microcatheter based at least in part on the location sensor in reference to the distal end of the guidewire.

The guidewire can have an outer diameter less than and approximately equal to an inner diameter of the lumen of the microcatheter.

The example transseptal puncturing system can further include a dilator having a lumen therethrough that has an inner diameter greater than and approximately equal to the inner diameter of the lumen of the microcatheter. The example transseptal puncturing system can further include a sheath configured to advance over the dilator. Alternatively, the microcatheter can have a tapered distal end and function as a dilator. When the system is configured as such, the tapered distal end of the microcatheter can have a distal outer diameter greater than and approximately equal to the outer diameter of the guidewire and a proximal outer diameter less than and approximately equal to the inner diameter of the lumen of the sheath.

The example transseptal puncturing system can further include a generator in electrical contact with the electrically conductive proximal end of the guidewire. The generator can be configured to provide electrical energy through the electrically conductive core of the guidewire to the distal end of the guidewire sufficient to puncture tissue so that the tissue is punctured without requiring a sharp end of a needle.

The example transseptal puncturing system can further include an impedance monitoring module in communication with the generator, in electrical communication with the proximal end of the guidewire, and configured to measure impedance at the distal end of the guidewire. The generator can be configured to provide the electrical energy to the distal end of the guidewire based at least in part on the impedance measured by the impedance monitoring module.

The steerable microcatheter can further include a pull wire configured to deflect the deflectable distal portion.

The location sensor can include a magnetic coil.

The location sensor can include an exposed electrode. The system can include one or more body patches. The navigation module can be configured to determine a position of the distal end of the microcatheter in the heart based at least in part on impedance between the body patches and the exposed electrode of the location sensor.

An example method for transseptal puncture can include one or more of the following steps performed in a variety of orders and with additional steps as understood by a person skilled in the pertinent art. A steerable microcatheter and a guidewire can be positioned within a right atrium such that an electrically conductive distal end of the guidewire is positioned approximate a distal end of the steerable microcatheter and exposed to blood. A position of the distal end of the microcatheter can be determined based at least in part on a location sensor of the microcatheter. The distal end of the microcatheter can be steered toward the target tissue to bring the distal end of the guidewire into contact with the target tissue. While the distal end of the guidewire is in contact with the target tissue, electrical energy can be applied through a conductive core of the guidewire, through the distal tip of the guidewire, and into the target tissue. While applying electrical energy, the guidewire can be advanced through the target tissue so that the distal end of the guidewire is in a left atrium.

The method can further include advancing a dilator and sheath over the guidewire and through the target tissue while the sheath is over the dilator. Alternatively, the microcatheter can be advanced over the guidewire, an opening through the target tissue can be dilated by the microcatheter, and a sheath can be advanced through the opening while the sheath is over the microcatheter.

The example method can further include mapping at least a portion of an interatrial septum using the location sensor.

The example method can further include determining the position of the distal end of the microcatheter based at least in part on the location sensor of the microcatheter in reference to the distal end of the guidewire.

The example method can further include measuring an impedance at the distal end of the guidewire. The example method can further include determining a position of the distal end of the guide wire based at least in part on the impedance. The example method can further include detecting contact with tissue based at least in part on the impedance. The example method can further include initiating application of the electrical energy based at least in part on the impedance. The example method can further include terminating the application of the electrical energy based at least in part on the impedance.

The example method can further include advancing the distal end of the microcatheter across the target tissue into the left atrium.

The example method can further include measuring a pressure of the left atrium through a lumen of the microcatheter.

The example method can further include injecting fluoroscopy contrast through a lumen of the microcatheter.

The example method can further include manipulating a pull wire of the microcatheter to deflect a compression coil, thereby steering the distal end of the microcatheter.

The location sensor can include an electromagnetic sensor. The location sensor can include three electromagnetic sensors.

The example method can further include determining a position of the distal end of the microcatheter in the heart based at least in part on impedance between a body patch and an exposed electrode of the location sensor.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of an example transseptal puncturing system according to aspects of the present invention.
FIG. 2A is an illustration of components of the example transseptal puncturing system having an alternative steerable microcatheter geometry according to aspects of the present invention.
FIG. 2B is an illustration of a cross section of components of the example transseptal puncturing system as indicated in FIG. 2A.
FIGs. 3A through 3I are a sequence of illustrations illustrating an example method for transseptal puncture according to aspects of the present invention.
FIGs. 4A through 4C are a sequence of illustrations illustrating alternative steps to the example method for transseptal puncture according to aspects of the present invention.
FIG. 5 is a flow diagram outlining steps of an example method for transseptal puncture according to aspects of the present invention.
FIG. 6 is an illustration of a transseptal perforation procedure according to aspects of the present invention.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

As used herein, the terms "component," "module," "system," "server," "processor," "memory," and the like are intended to include one or more computer-related units, such as but not limited to hardware, firmware, a combination of hardware and software, software, or software in execution. For example, a component may be, but is not limited to being, a process running on a processor, an object, an executable, a thread of execution, a program, and/or a computer. By way of illustration, both an application running on a computing device and the computing device can be a component. One or more components can reside within a process and/or thread of execution and a component may be localized on one computer and/or distributed between two or more computers. In addition, these components can execute from various computer readable media having various data structures stored thereon. The components may communicate by way of local and/or remote processes such as in accordance with a signal having one or more data packets, such as data from one component interacting with another component in a local system, distributed system, and/or across a network such as the Internet with other systems by way of the signal. Computer readable medium can be non-transitory. Non-transitory computer-readable media include, but are not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable instructions and/or data.

As used herein, the term "computing system" is intended to include stand-alone machines or devices and/or a combination of machines, components, modules, systems, servers, processors, memory, detectors, user interfaces, computing device interfaces, network interfaces, hardware elements, software elements, firmware elements, and other computer-related units. By way of example, but not limitation, a computing system can include one or more of a general-purpose computer, a special-purpose computer, a processor, a portable electronic device, a portable electronic medical instrument, a stationary or semi-stationary electronic medical instrument, or other electronic data processing apparatus.

As used herein, the term "microcatheter" is a catheter having a diameter that is small in comparison to catheters in cardiovascular applications, i.e. 8 French or less.

As used herein, the term "needle" describes a structure having a sharp pointed end designed to puncture tissue.

As used herein, the term "non-transitory computer-readable media" includes, but is not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable information.

As used herein, the term "radiofrequency" (RF) is used to refer to an alternating current that flows through a conductor.

As used herein, the terms "tubular" and "tube" are to be construed broadly and are not limited to a structure that is a right cylinder or strictly circumferential in cross-section or of a uniform cross-section throughout its length. For example, a tubular structure or system is generally illustrated as a substantially right cylindrical structure. However, the tubular system may have a tapered or curved outer surface without departing from the scope of the present disclosure.

In current transseptal puncture procedures, it can be difficult to achieve desired precision in location and angle of puncture. A bulky sheath and dilator can be difficult to precisely position, and a stiff needle can deflect the sheath and dilator as the needle is moved distally through the sheath and dilator and when forced against tissue. In some examples illustrated herein, an example transseptal puncturing system can be used to more safely and/or more precisely perform a transseptal puncture. In some examples the transseptal puncturing system can be less bulky, nimbler, and/or require less puncturing force than many current transseptal puncturing systems.

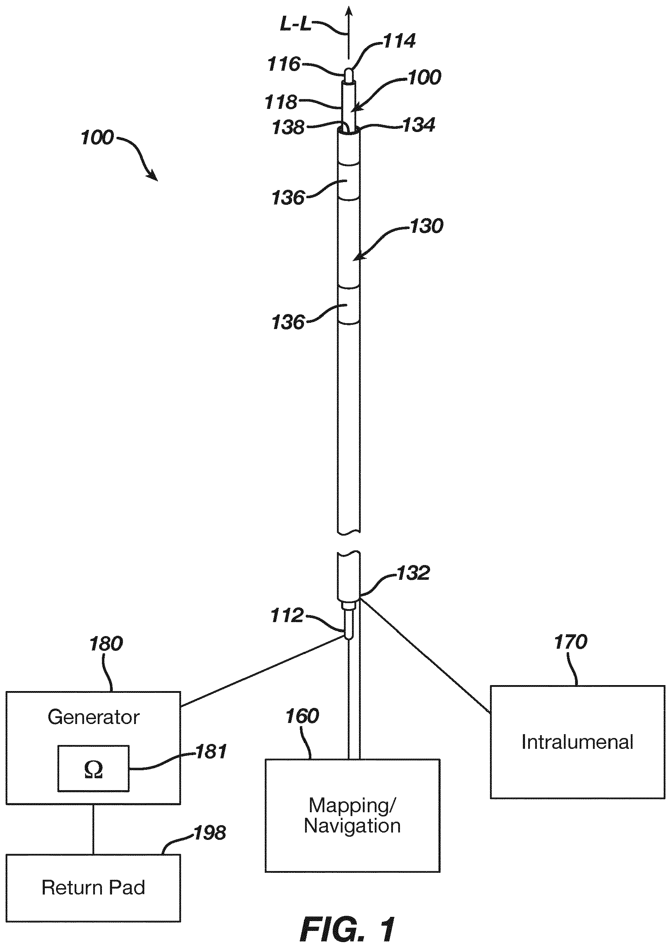

FIG. 1 is an illustration of an example transseptal puncturing system 100. The system 100 includes a guidewire 110, a microcatheter 130, a generator 180, a return pad 198, a mapping/navigation module 160, and an intralumenal module 170.

The guidewire 110 can have a solid conductive core 116 and an insulating jacket 118. A distal end 114 and a proximal end 112 of the guidewire 110 can each be non-insulated so that electrical contact can be made from the proximal end 112 through the conductive core 116 to the distal end 114. Alternatively, the guidewire 110 need not include the insulating jacket 118.

The generator 180 can be electrically connected to the proximal end 112 of the guidewire 110 through an easily attachable connector/cable and can provide electrical signals through the core 116 of the guidewire 110 to the distal end 114 of the guidewire that are sufficient to puncture tissue, using RF energy, without requiring a needle or sharp end. The generator 180 can provide RF signals to the distal end 114 of the guidewire 110 that spread from the distal end 114 through a body of a patient to the return pad 198. The generator 180 can include an impedance monitoring module 181 that is configured to measure impedance at the distal end 114 of the guidewire 110 to help facilitate a use case. The generator 180 can be configured to provide electrical energy to the distal end 114 of the guidewire 110 based at least in part on the impedance measured by the impedance monitoring module 181. Additionally, or alternately, the generator 180 can be configured to provide electrical energy to the distal end 114 of the guidewire 110 based on a fixed predetermined time.

The microcatheter 130 has a lumen 138 in which the guidewire 110 is positioned. The microcatheter 130 is aligned along a longitudinal axis L-L. The microcatheter 130 can have a deflectable distal portion near a distal end 134 of the microcatheter 130. The microcatheter 130 can have one or more location sensors 136 positioned on the distal portion. The location sensor(s) 136 can include one or more magnetic coils and/or one or more impedance sensors.

The mapping/navigation module 160 can be in electrical contact with the location sensor(s) 136, for example via one or more electrical conductors extending longitudinally through the microcatheter 130 from the location sensors 136 to a proximal end 132 of the microcatheter 130. The navigation module 160 can be configured to determine a position and/or orientation of the distal portion of the microcatheter 130 based at least in part on electrical signals from the location sensor(s) 136 of the microcatheter 130. The navigation module 160 can include an electric tracking sub-system and/or a magnetic position tracking sub-system which can function alone or in a hybrid mode as described in U.S. 8,456,182 incorporated herein by reference and attached in the Appendix to priority application U.S. 63/190,865 or as otherwise understood by a person skilled in the pertinent art. The navigation module 160 can further be in electrical contact with the proximal end 112 of the guidewire 110 and can utilize the distal end 114 of the guidewire as a reference electrode for the location sensor(s) 136.

Additionally, or alternatively, the guidewire 110 can include one or more location sensors configured as described in relation to location sensor 136.

The intralumenal module 170 can be in communication with the lumen 138 of the microcatheter 130. The intralumenal module 170 can be configured to perform intralumenal steps as understood by a person skilled in the pertinent art such as sensing pressure and/or providing fluids via the lumen 138 of the microcatheter 130.

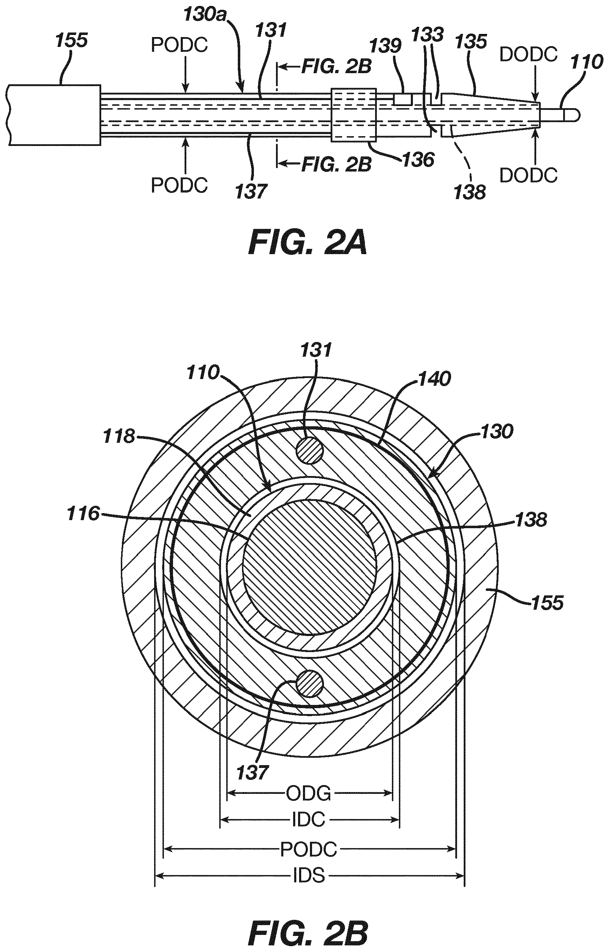

FIG. 2A is an illustration of components of the example transseptal puncturing system 100 illustrated in FIG. 1 with an exception that the steerable microcatheter 130a has a tapered distal portion 135. The microcatheter 130a illustrated in FIG. 2A can be shaped and otherwise configured to function as a dilator.

FIG. 2B is an illustration of a cross section of the system 100 as indicated in FIG. 2A.

Referring collectively to FIGs. 2A and 2B, the microcatheter 130a can have a distal outer diameter (DODC) that is greater than, and approximately equal to, an outer diameter of the guidewire (ODG). The microcatheter 130a can have a proximal outer diameter (PODC) that is less than and approximately equal to an inner diameter of the sheath (IDS). The microcatheter 130 illustrated in FIG. 1 can be configured similarly to the microcatheter 130a illustrated in FIGs. 2A and 2B with an exception that the proximal outer diameter (PODC) is smaller as the microcatheter 130 need not function as a dilator in some applications. The microcatheter 130, 130a can include a pull wire 131 that can be pulled to deflect a distal portion of the microcatheter 130, 130a from the longitudinal axis L-L. The pull wire 131 can be anchored to the body of the microcatheter 130a by a pull wire anchor 139. The microcatheter 130, 130a can include one or more sensor wires 137 electrically connected to the location sensor 136. The microcatheter 130, 130a can include a braid layer 140. The microcatheter 130, 130a can include one or more fluidic/irrigation ports 133. The tapered distal end 135 of the microcatheter 130a can be fused to a shaft of the microcatheter 130a.

The guidewire 110 can include an insulative jacket 118 over a majority of the conductive core 116 of the guidewire 110. The insulative jacket 118 can define the outer diameter (ODG) of the guidewire 110. When the guidewire 110 lacks an insulative jacket 118, the conductive core 116 can define the outer diameter (ODG) of the guidewire 110. The microcatheter 130, 130a can have inner diameter (IDC) within the lumen 138 of the microcatheter 130, 130a that is greater than and approximately equal to the outer diameter (ODG) of the guidewire 110. The lumen 138 of the microcatheter 130, 130a can be tapered such that the inner diameter (IDC) of the microcatheter 130, 130a is smaller at a distal end of the microcatheter 130, 130a. Dimensions of the outer diameter (ODG) of the guidewire 110 and inner diameter (IDC) of the microcatheter 130, 130a can be sized such that the guidewire 110 is snugly held in the lumen 138 of the microcatheter 130, 130a so that the manipulation of the microcatheter 130, 130a precisely controls positioning of the distal end 114 of the guidewire 110. Preferably, the dimensions of the outer diameter (ODG) of the guidewire 110 and inner diameter (IDC) of the microcatheter 130, 130a as sized to allow longitudinal translation of the guidewire 110 within the lumen 138 of the microcatheter 130, 130a. The microcatheter 130, 130a can have an outer diameter (ODG) of about 8 French or less. The guidewire 110 preferably has an outer diameter (ODG) of about 0.032 inches (0.81 millimeters) to about 0.014 inches (0.36 millimeters). The microcatheter 130, 130a preferably has an inner diameter (IDC) of about 0.036 inches (0.91 millimeters) to about 0.04 inches (1 millimeter).

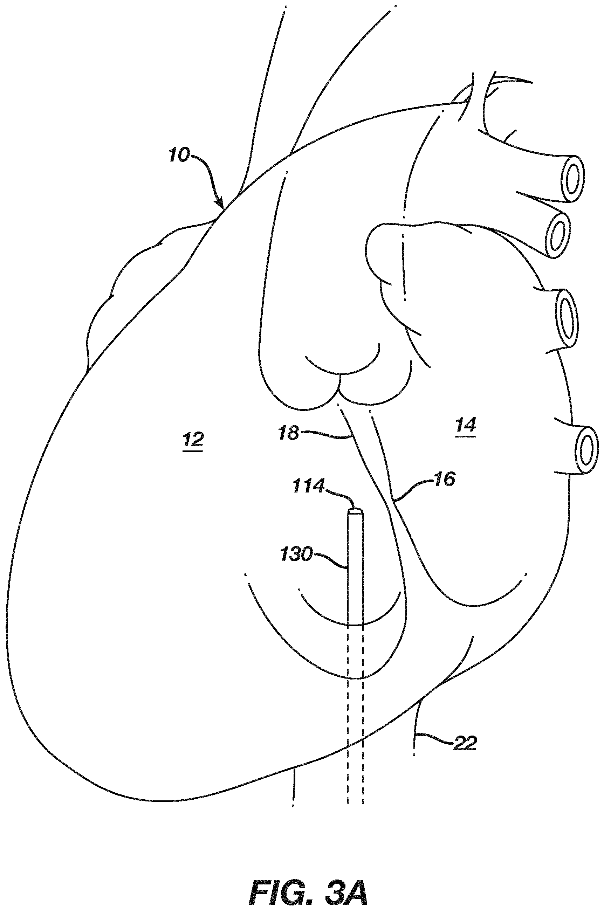

FIGs. 3A through 3H are a sequence of illustrations illustrating an example method for transseptal puncture. FIGs. 3A through 3H are illustrated with the low profile steerable microcatheter 130 illustrated in FIG. 1. FIGs. 3A through 3G can also be carried out in a similar manner with the steerable microcatheter 130a having a tapered distal portion 135 as illustrated in FIGs. 2A and 2B.

FIG. 3A illustrates the microcatheter 130 and guidewire 110 traversed through the inferior vena cava 22 such that a distal portion of the microcatheter 130 and the guidewire 110 is positioned within the right atrium 12. The distal end 114 of the guidewire 110 is exposed to blood.

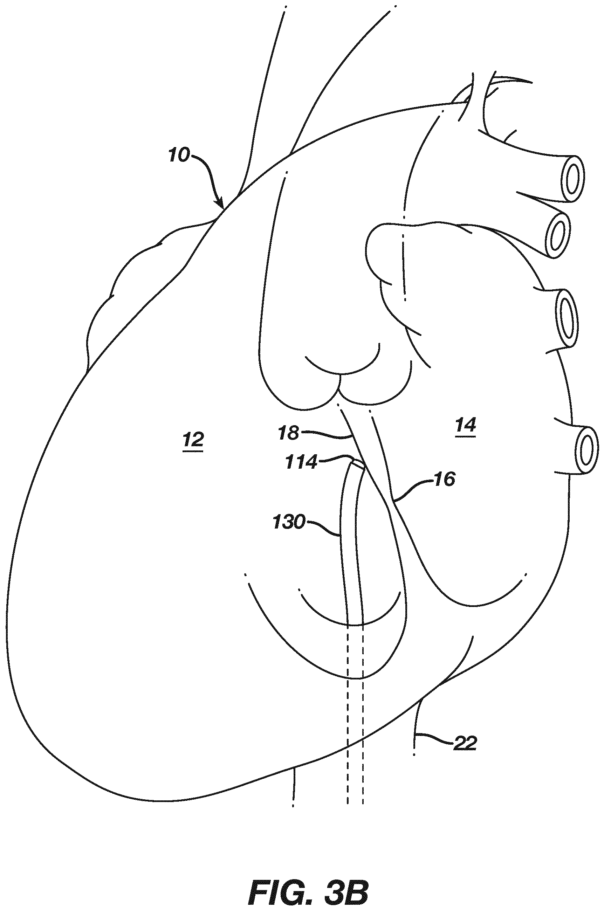

FIG. 3B illustrates the distal portion of the microcatheter 130 being deflected. In some procedures, the microcatheter 130 can be deflected and moved about the right atrium 12 to map the right atrium. The navigation/mapping module 160 can receive electrical signals from the location sensor(s) 136 of the microcatheter to map portions of the right atrium 12. For instance, the microcatheter 130 can be manipulated to map a portion of a septal wall 18 in the right atrium 12 to locate a fossa ovalis 16 and the ideal placement for the puncture site particular to the procedure being performed. In some examples, the navigation/mapping module 160 can receive electrical signals from the electrically conductive distal end 114 of the guidewire 110 and electrical signals from the location sensor(s) 136 such that the distal end 114 of the guidewire 110 is used as a reference electrode to the location sensor(s) 136.

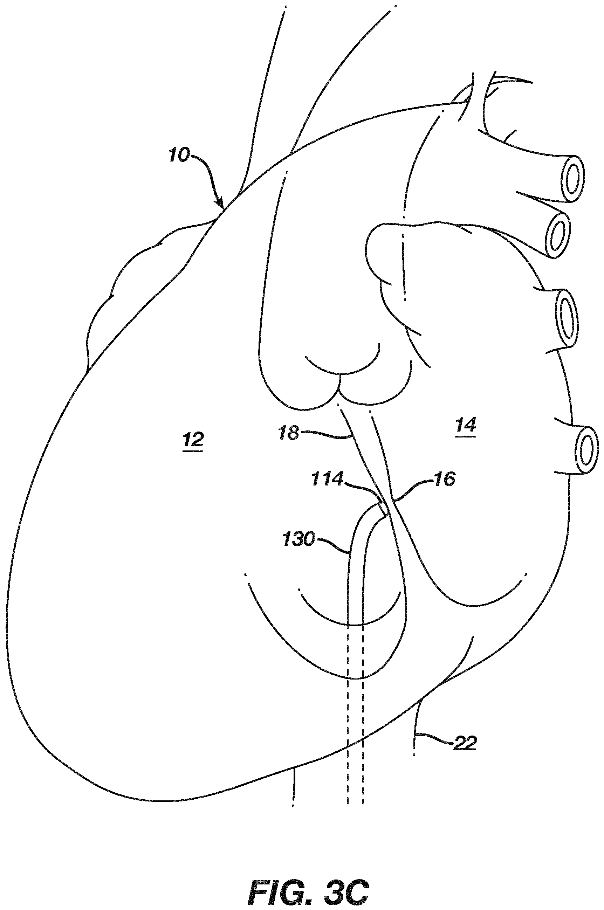

FIG. 3C illustrates the microcatheter 130 being manipulated to position the electrically conductive distal end 114 of the guidewire 110 against target tissue, i.e. the septal wall 18 at the fossa ovalis 16.

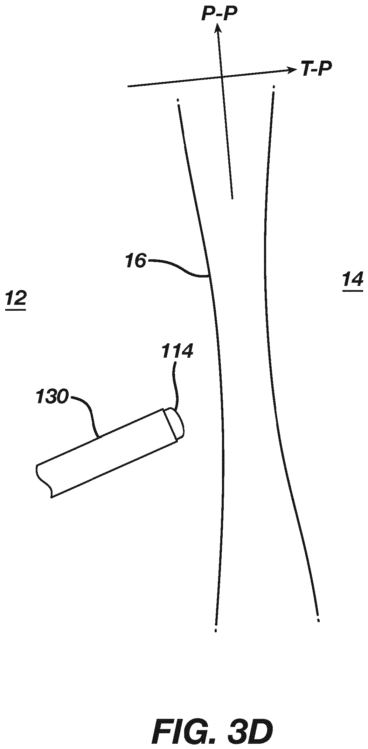

FIG. 3D illustrates a zoomed in view of the electrically conductive distal end 114 of the guidewire 110 approaching the fossa ovalis 16. As oriented in the illustration, the fossa ovalis 16 aligns with a parallel axis P-P and is orthogonal to a traverse axis T-P. The distal portion of the microcatheter 130 can repositioned and deflected to a desired position on the fossa ovalis 16 and a desired angle of approach relative to the parallel axis P-P and traverse axis T-P.

As the electrically conductive distal end 114 of the guidewire 110 approaches tissue of the fossa ovalis 16, impedance at the distal end 114 can be monitored by the impedance monitoring module 181. When the distal end 114 of the guidewire is far enough from tissue so that it is known through mapping, fluoroscopy, or other means to be in contact with blood, a pre-contact impedance can be sensed by the impedance monitoring module 181. When the distal end 114 of the guidewire 110 comes into contact with tissue, a tissue impedance can be sensed by the impedance monitoring module 181. Contact of the distal end 114 of the guidewire 110 to the tissue can be detected by the impedance monitoring module 181 as a change in impedance from the pre-contact impedance to the tissue impedance as well as the impedance post penetration of the fossa ovalis tissue 16.

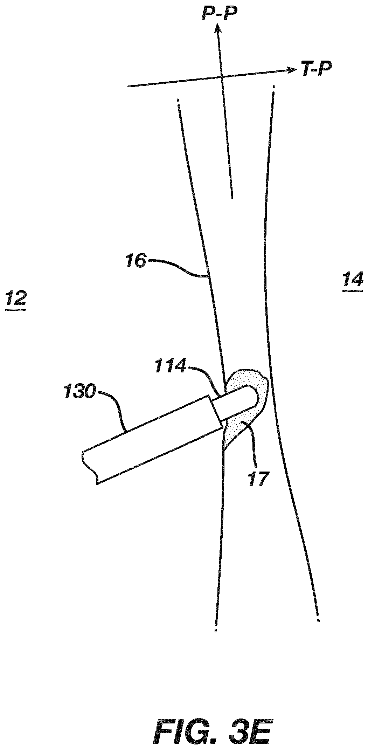

FIG. 3E illustrates the distal end 114 of the guidewire 110 positioned within tissue. Electrical energy can be applied by the generator 180 to the electrically conductive distal end 114 of the guidewire 110 to ablate or otherwise create damaged tissue 17 around the distal end 114 of the guidewire 110.

Mechanically, the distal end 114 of the guidewire can be atraumatic, i.e. lacking a traumatic or sharp end such that absent application of the electrical energy from the generator 180, the guidewire 110 is unable to puncture tissue. The system 100 need not require a needle to puncture tissue. The distal end 114 of the guidewire 110 can have a hemispherical shape as illustrated, a flat shape, a domed shape, or other such atraumatic shape. In contrast, U.S. Pat. No. 10,413,707 (incorporated herein by reference and attached to the Appendix to priority application U.S. 63/190,865) discloses a guidewire with a piercing end configured to mechanically pierce tissue absent application of electrical energy. The guidewire 110 of the present disclosure need not have such piercing end to puncture tissue because tissue puncture can be achieved by application of electrical energy to the distal end 114 of the guidewire 110.

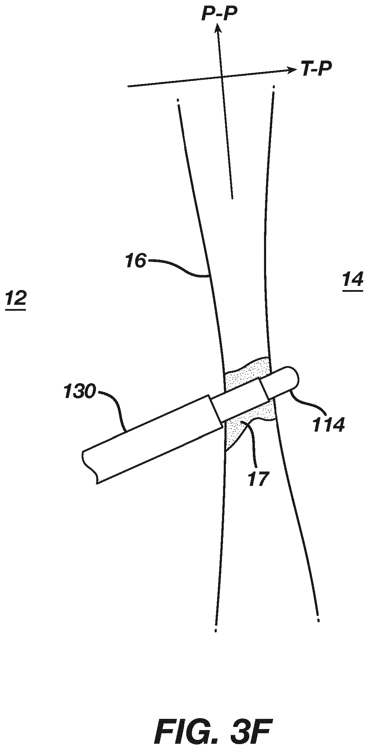

FIG. 3F illustrates the distal end 114 of the guidewire 110 exiting the tissue into the left atrium 14. In some examples, tissue impedance can be monitored by the impedance monitoring module 181 while the distal end 114 of the guidewire 110 travels through the tissue. A change in impedance from the tissue impedance to the post-contact impedance sensed by the impedance monitoring module 181 can indicate that the distal end 114 has entered the left atrium 14. The generator 180 can cease application of electrical energy in response to the change in impedance detected by the impedance monitoring module 181 when the distal end 114 enters the left atrium 14.

Additionally, or alternatively the generator 180 can cease application of electrical energy in response to time elapsed from a start time. The time at which the distal end 114 of the guidewire initially contacts the tissue of the fossa ovalis 16 can be sensed by the impedance monitoring module 181 and recorded or otherwise utilized as the start time used as a reference for when to terminate application of electrical energy from the generator 180. Alternatively, the start time can be determined based on initial application of electrical energy from the generator 180 to the distal end 114 of the guidewire 110. Supply of electrical energy from the generator 180 can be terminated when a predetermined time has elapsed following the start time. In some examples, the predetermined time can be set to as high as about 2 seconds; however, during most treatments, crossing can be completed within 1 millisecond, and therefore the predetermined time can be set at about 1 milliseconds to about 10 milliseconds.

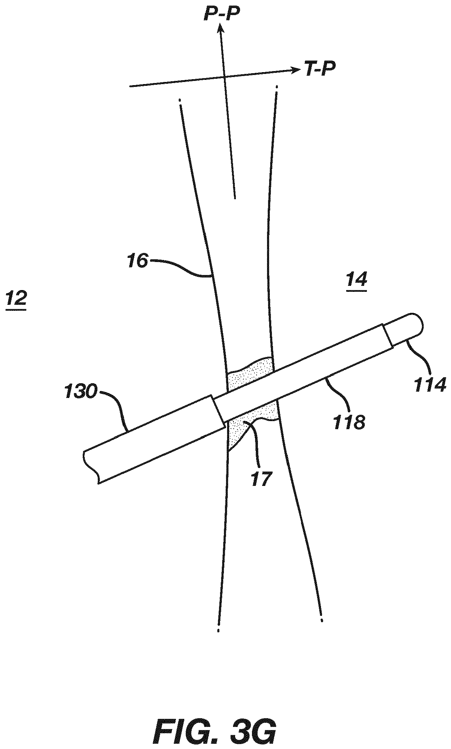

FIG. 3G illustrates the guidewire 110 being further moved distally into the left atrium 14. At the illustrated instance, the electrical energy from the generator 180 is preferably ceased due to change in impedance and/or elapse of time as disclosed above; however, in a treatment in which the electrical energy remains applied from the generator 180, the insulative jacket 118 over the guidewire 110 can inhibit further damage to tissue of the fossa ovalis 16. Alternatively, the energy from the generator 180 can be precisely controlled to shut off before tissue is overly damaged, in which case the insulative jacket 118 is not necessary to inhibit further damage to tissue.

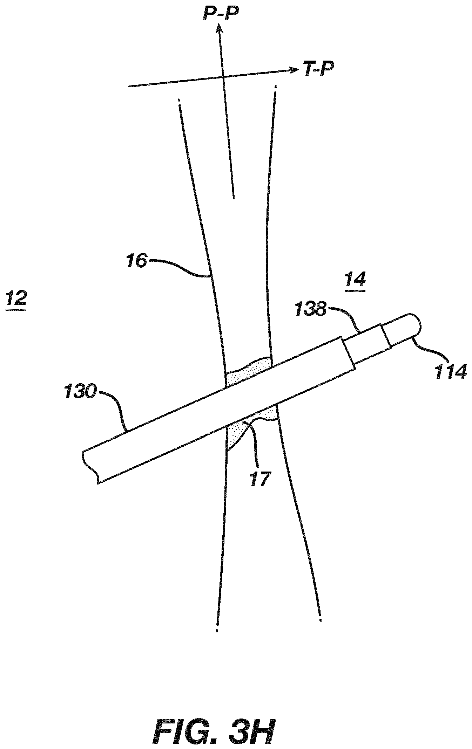

FIG. 3H illustrates an optional step in which the microcatheter 130 crosses the fossa ovalis 16. While positioned as illustrated, the intralumenal module 170 can perform intralumenal steps as understood by a person skilled in the pertinent art such as sensing pressure and/or providing fluids via the lumen 138 of the microcatheter 130.

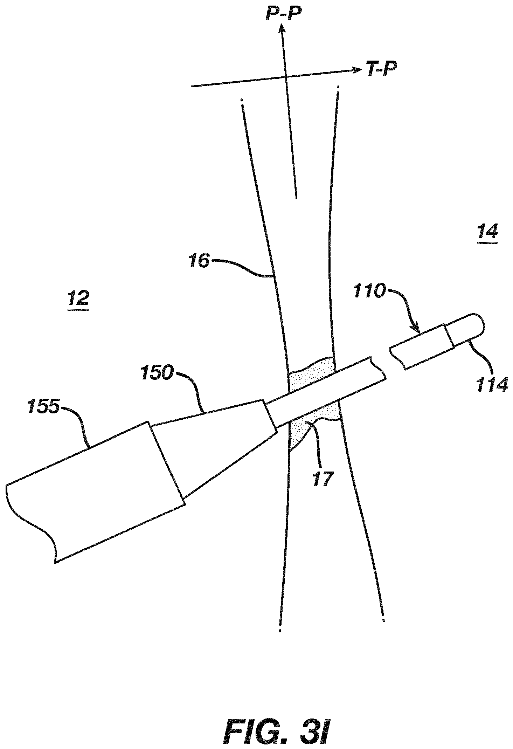

FIG. 3I illustrates a dilator 150 and sheath 155 moved distally over the guidewire 110 to the transseptal puncture through the fossa ovalis 16. The microcatheter 130 can be removed prior to this step (as illustrated), or alternatively, the microcatheter 130 can be left in place so that the dilator 150 and sheath 155 are moved distally over the microcatheter 130. The dilator 150 and sheath can be moved through the transseptal puncture, and treatment within the left atrium 14 can proceed according to various methods as understood by a person skilled in the pertinent art.

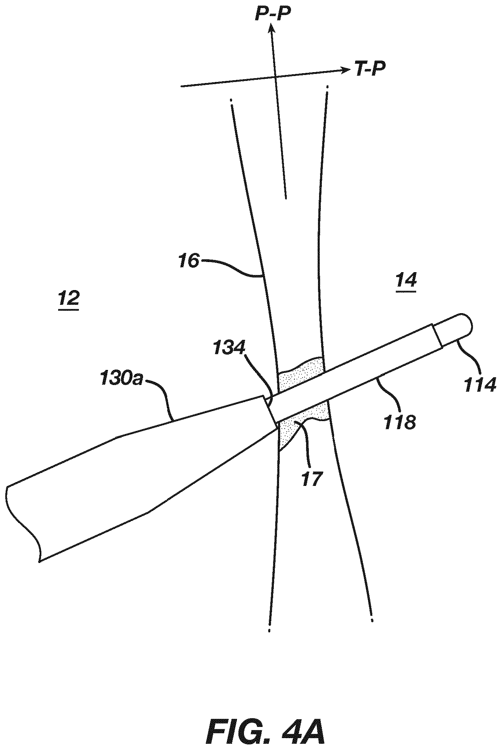

FIGs. 4A through 4C are a sequence of illustrations which can be performed in place of the steps illustrated in FIGs. 3H and 3I when the steerable microcatheter 130a is tapered to function as a dilator as illustrated in FIG. 2A.

FIG. 4A illustrates the tapered dilator positioned entirely in the right atrium 12 such that the distal end 134 is positioned at the fossa ovalis immediately following the step described in relation to FIG. 3G.

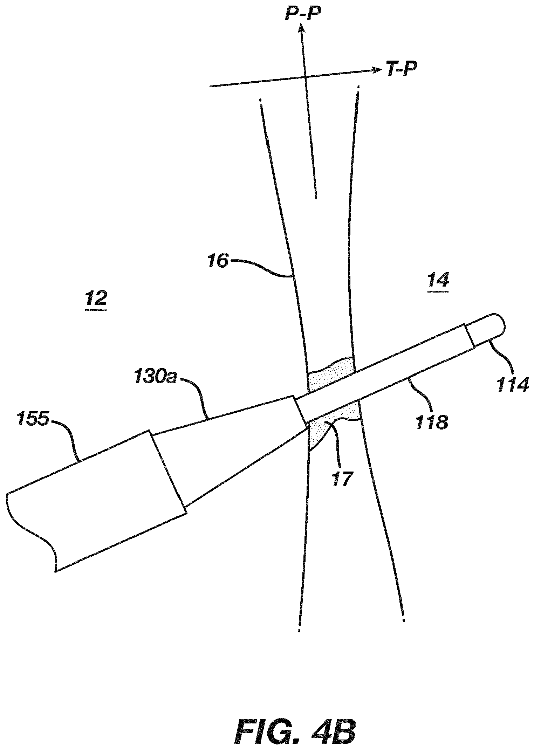

FIG. 4B illustrates the sheath 155 being moved over the microcatheter 130a.

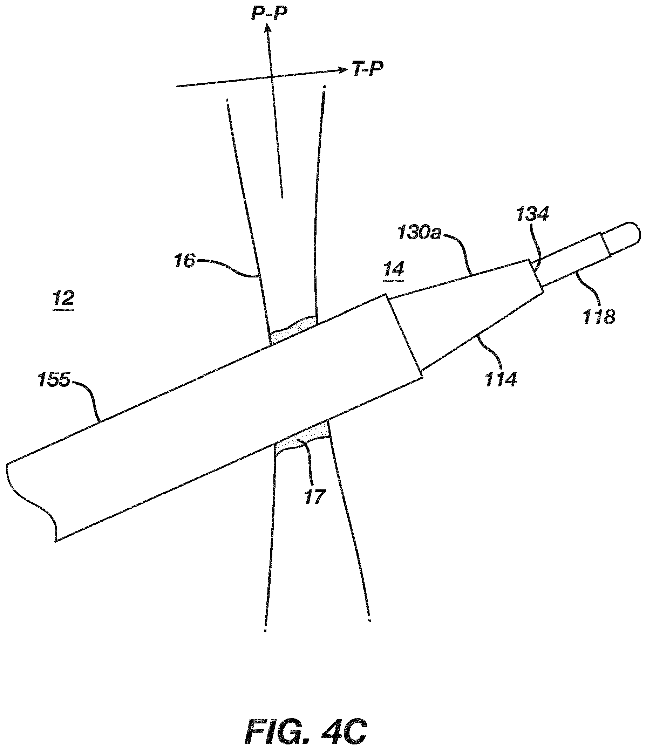

FIG. 4C illustrates the microcatheter 130a dilating the opening in the fossa ovalis 16 as the distal end 134 of the microcatheter 130a enters the left atrium 14. While positioned as illustrated, the intralumenal module 170 can perform intralumenal steps as understood by a person skilled in the pertinent art such as sensing pressure and/or providing fluids via the lumen 138 of the microcatheter 130.

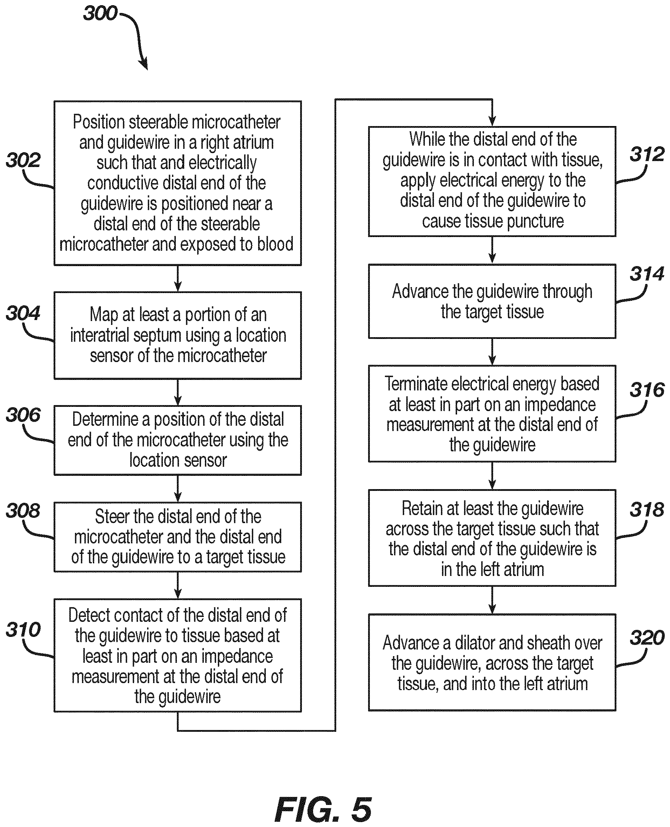

FIG. 5 is a flow diagram outlining steps of an example method 300 for transseptal puncture. At step 302, a steerable microcatheter and guidewire can be positioned in a right atrium such that an electrically conductive distal end of the guidewire is positioned near a distal end of the steerable microcatheter and exposed to blood. The microcatheter and guidewire can be configured similarly to the microcatheter 130 and the guidewire 110 disclosed herein, variations thereof, and alternatives thereto as understood by a person skilled in the pertinent art.

At step 304, at least a portion of an interatrial septum can be mapped using a location sensor of the microcatheter. The location sensor can be configured similarly to the location sensor(s) 136 disclosed herein, variations thereof, and alternatives thereto as understood by a person skilled in the pertinent art.

At step 306, a position of the distal end of the microcatheter can be determined using the location sensor.

At step 308, the distal end of the microcatheter can be steered to thereby steer the distal end of the guidewire to target tissue.

At step 310, contact of the distal end of the guidewire to tissue can be detected based at least in part on an impedance measurement at the distal end of the guidewire.

At step 312, while the distal end of the guidewire is in contact with tissue, electrical energy can be applied to the distal end of the guidewire to cause tissue puncture.

At step 314, the guidewire can be advanced through the target tissue. The microcatheter can also be advanced through the target tissue, preferably after the electrical energy to the guidewire is terminated

At step 316, the electrical energy at the distal end of the guidewire can be terminated based at least in part on an impedance measurement at the distal end of the guidewire.

At step 318, the guidewire can be retained across the target tissue such that the distal end of the guidewire is in the left atrium. The microcatheter may also be retained across the target tissue.

At step 320, a dilator and a sheath can be advanced over the guidewire, across the target tissue, and into the left atrium. If the microcatheter is retained across the target tissue, the dilator and sheath can be advanced over the microcatheter, across the target tissue, and into the left atrium.

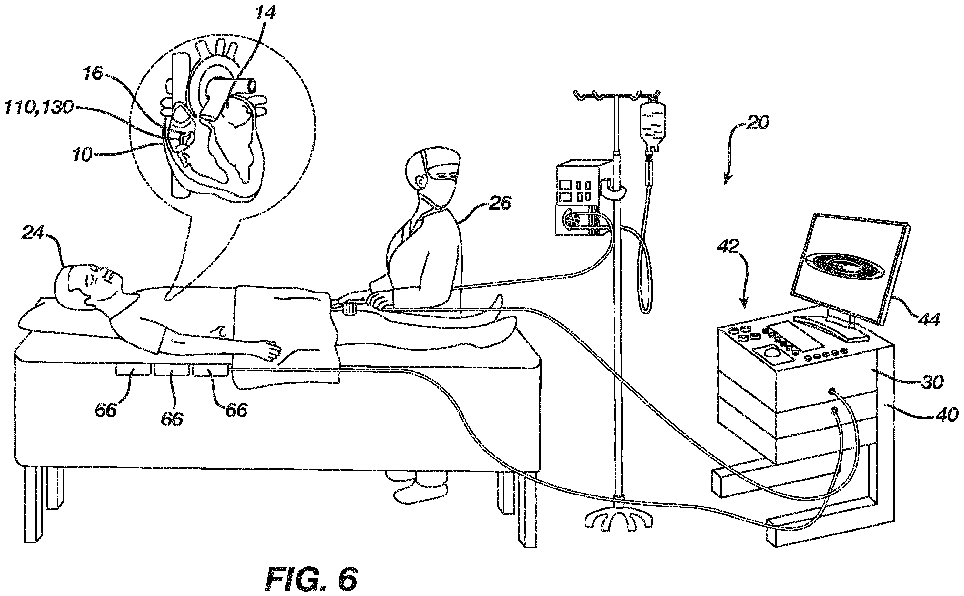

FIG. 6 is an illustration of a transseptal perforation procedure using a computer-aided system 20. The system 20 can be used during a medical procedure on a heart 10 of a patient 24 to perform transseptal perforation. The procedure can be performed by one or more operators including a medical professional 26. The system 20 can be configured to present images of a cavity, such as an internal chamber of heart 10, allowing operator 26 to visualize characteristics of the cavity. The system 20 can further be configured to present images of the microcatheter 130 and/or guidewire 110. The system 20 can further include and/or be configured to control components of the transseptal perforation system 100 illustrated in FIG. 1.

The system 20 can be controlled by a system processor 30 which can be realized as a general-purpose computer. The processor 30 can be mounted in a console 40. The console 40 can include operating controls 42 such as a keypad and a pointing device such as a mouse or trackball that the operator 26 can use to interact with the processor 30. Results of the operations performed by the processor 30 can be provided to the operator on a display 44 connected to the processor 30. The display 44 can further present a graphic user interface to the operator enabling the operator to control the system 20. The operator 26 may use controls 42 to input values of parameters used by the processor 30 in the operation of the system 20.

The processor 30 uses computer software to operate the system 20. The software can be downloaded to the processor 30 in electronic form, over a network, for example, or it can, alternatively or additionally, be provided and/or stored on non-transitory tangible computer-readable media, such as magnetic, optical, or electronic memory.

The operator 26 can insert the microcatheter 130 and guidewire 110 into the patient 24, so that a distal end of the microcatheter 130 and guidewire 110 enters right atrium 12 of the patient's heart via the inferior vena cava 22. The processor 30 can be configured to track the distal end 134 of the microcatheter and the distal end 114 of the guidewire 110, typically both the location and the orientation of the distal ends 114, 134, while they are within heart 10. When the sensor(s) 136 of the microcatheter 130 include one or more magnetic coil(s), the processor 30 can utilize a magnetic tracking system such as is provided by the Carto^{®} system produced by Biosense Webster. The system 20 can include magnetic field transmitters 66 in the vicinity of patient 24, so that magnetic fields from the transmitters interact with magnetic coil(s) near the distal end 134 of the microcatheter 130. The coils interacting with the magnetic fields generate signals which are transmitted to the processor 30, and the processor analyzes the signals to determine the location and orientation of the guidewire 110 and microcatheter 130.

Additionally, or alternative, the system 20 can include body patches (not illustrated) for an electrical tracking sub-system that is impedance based, also referred to as an advanced current localization (ACL) tracker sub-system. In the ACL sub-system, current is delivered to an impedance sensor that is within the patient's body, the current spreads from the impedance sensor through the body to the body patches, and location of the impedance sensor is calculated based on current distribution at the body patches. The sensor(s) 136 of the microcatheter 130 can include one or more impedance sensors, and/or the electrically conductive distal end 114 of the guidewire 110 can function as an impedance sensor.

The system 20 can include a magnetic tracking sub-system and/or an ACL sub-system configured similarly to as disclosed in U.S. 8,456,182, which is incorporated herein by reference and attached in the Appendix to priority application U.S. 63/190,865.

The console 40 can further be configured to monitor impedance and/or control electrical energy to guidewire 110 as illustrated and described elsewhere herein.

The descriptions contained herein are examples of embodiments of the invention and are not intended in any way to limit the scope of the invention. As described herein, the invention contemplates many variations and modifications of system components, including alternative tip shapes, alternative numbers of electrodes, alternative location sensors, combinations of components illustrated in separate figures, alternative materials, alternative component geometries, and alternative component placement. Modifications and variations apparent to those having skilled in the pertinent art according to the teachings of this disclosure are intended to be within the scope of the claims which follow.

### EMBODIMENTS

1. A transseptal puncturing system comprising:
   a steerable microcatheter comprising an elongated member with a lumen extending therethrough to define a longitudinal axis, a deflectable distal portion, and a location sensor disposed proximate the deflectable distal portion;
   a guidewire disposed within the lumen of the microcatheter comprising an electrically conductive core, an electrically conductive distal end, an electrically conductive proximal end, and an outer diameter less than and approximately equal to an inner diameter of the lumen of the microcatheter; and
   a generator in electrical contact with the electrically conductive proximal end, the generator being configured to provide electrical energy to the distal end of the guidewire sufficient to puncture tissue without requiring a sharp end.
2. The system of embodiment 1, further comprising:
   a navigation module configured to determine a position of the distal end of the microcatheter in a heart based at least in part on the location sensor in reference to the electrically conductive distal end of the guidewire.
3. The system of embodiment 1, further comprising:
   a dilator comprising a lumen therethrough comprising an inner diameter greater than and approximately equal to the inner diameter of the lumen of the microcatheter; and
   a sheath configured to advance over the dilator.
4. The system of embodiment 1, wherein the steerable microcatheter further comprises a pull wire connected to the distal portion to deflect the distal portion with respect to the longitudinal axis.
5. The system of embodiment 1, further comprising:
   a mapping module configured to generate a map of at least a portion of an interatrial septum using the location sensor.
6. A transseptal puncturing system comprising:
   a steerable microcatheter having an elongated tubular member with a lumen extending therethrough to define a longitudinal axis, a deflectable distal portion, and a location sensor disposed proximate the distal portion along the longitudinal axis; and
   a guidewire having a portion disposed in the lumen of the microcatheter, the guidewire comprising an electrically conductive core, an electrically conductive distal end, and an electrically conductive proximal end; and
   a navigation module configured to determine a position of the distal end of the microcatheter based at least in part on the location sensor in reference to the distal end of the guidewire.
7. The system of embodiment 6, wherein the guidewire comprises an outer diameter less than and approximately equal to an inner diameter of the lumen of the microcatheter.
8. The system of embodiment 7, further comprising:
   a dilator comprising a lumen therethrough comprising an inner diameter approximately equal to the inner diameter of the lumen of the microcatheter; and
   a sheath configured to advance over the dilator.
9. The system of embodiment 1 or embodiment 6, further comprising:
   a sheath configured to advance over the microcatheter,
   wherein the microcatheter comprises a tapered distal end, and
   wherein the tapered distal end comprises a distal outer diameter greater than and approximately equal to the outer diameter of the guidewire and a proximal outer diameter less than and approximately equal to inner diameter of a lumen of the sheath.
10. The system of embodiment 6, further comprising:
   a generator in electrical contact with the electrically conductive proximal end, the generator being configured to provide electrical energy to the distal end of the guidewire sufficient to puncture tissue.
11. The system of embodiment 1 or embodiment 10, further comprising:
   an impedance monitoring module in communication with the generator, in electrical communication with the proximal end of the guidewire, and configured to measure impedance at the distal end of the guidewire,
   wherein the generator is configured to provide the electrical energy to the distal end of the guidewire based at least in part on the measured impedance.
12. The system of embodiment 6, wherein the steerable microcatheter further comprises a pull wire configured to deflect the deflectable distal portion.
13. The system of embodiment 1 or embodiment 6, wherein the location sensor comprises a magnetic coil.
14. The system of embodiment 1 or embodiment 6, wherein the location sensor comprises an exposed electrode.
15. The system of embodiment 14, further comprising:
   a body patch; and
   a navigation module configured to determine a position of the distal end of the microcatheter in a heart based at least in part on impedance between the body patch and the exposed electrode of the location sensor.

## Claims

1. A transseptal puncturing system comprising:
a guidewire comprising an electrically conductive core, an electrically conductive distal end, and an electrically conductive proximal end;
a generator in electrical contact with the electrically conductive proximal end, the generator being configured to provide electrical energy to the distal end of the guidewire sufficient to puncture tissue without requiring a sharp end;
a navigation module configured to determine a position of the distal end of the guidewire in a heart; and
an impedance monitoring module in communication with the generator, in electrical communication with the proximal end of the guidewire, and configured to measure impedance at the distal end of the guidewire,
wherein the generator is configured to provide the electrical energy to the distal end of the guidewire based at least in part on the measured impedance.

2. The transseptal puncturing system of claim 1, further comprising:
a steerable microcatheter comprising an elongated member with a lumen extending therethrough to define a longitudinal axis, a deflectable distal portion, and a location sensor disposed proximate the deflectable distal portion,
wherein the guidewire is disposed within the lumen of the microcatheter, wherein the guidewire has an outer diameter less than and approximately equal to an inner diameter of the lumen of the microcatheter, and
wherein the navigation module is further configured to determine a position of the distal end of the microcatheter in a heart based at least in part on the location sensor in reference to the electrically conductive distal end of the guidewire.

3. The system of claim 2, further comprising:
a dilator comprising a lumen therethrough comprising an inner diameter greater than and approximately equal to the inner diameter of the lumen of the microcatheter; and
a sheath configured to advance over the dilator.

4. The system of claim 2, wherein the steerable microcatheter further comprises a pull wire connected to the distal portion to deflect the distal portion with respect to the longitudinal axis.

5. The system of claim 2, further comprising:
a mapping module configured to generate a map of at least a portion of an interatrial septum using the location sensor.

6. The system of claim 2, further comprising:
a sheath configured to advance over the microcatheter,
wherein the microcatheter comprises a tapered distal end, and
wherein the tapered distal end comprises a distal outer diameter greater than and approximately equal to the outer diameter of the guidewire and a proximal outer diameter less than and approximately equal to inner diameter of a lumen of the sheath.

7. The system of claim 2, wherein the location sensor comprises a magnetic coil.

8. The system of claim 2, wherein the location sensor comprises an exposed electrode.

9. The system of claim 8, further comprising:
a body patch; and
a navigation module configured to determine a position of the distal end of the microcatheter in a heart based at least in part on impedance between the body patch and the exposed electrode of the location sensor.
